(19) Europäisches Patentamt
European Patent Office
Office européen des brevets

(11) **EP 4 104 703 A1**

(12) **EUROPEAN PATENT APPLICATION**
published in accordance with Art. 153(4) EPC

(43) Date of publication:
**21.12.2022 Bulletin 2022/51**

(21) Application number: **21754357.8**

(22) Date of filing: **08.02.2021**

(51) International Patent Classification (IPC):
**A44B 18/00** (2006.01)   **A41C 1/00** (2006.01)
**A41C 1/02** (2006.01)   **A41D 13/05** (2006.01)

(52) Cooperative Patent Classification (CPC):
**A41C 1/00; A41C 1/02; A41D 13/05; A44B 18/00**

(86) International application number:
**PCT/JP2021/004572**

(87) International publication number:
**WO 2021/161955 (19.08.2021 Gazette 2021/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.02.2020 JP 2020021217**

(71) Applicant: **Toray Industries, Inc.**
**Tokyo, 103-8666 (JP)**

(72) Inventors:
• **TAKADA, Nana**
**Otsu-shi, Shiga 520-2141 (JP)**
• **HANANOUCHI, Hirotaka**
**Otsu-shi, Shiga 520-2141 (JP)**
• **MIYAMURA, Takako**
**Tokyo 101-0032 (JP)**

(74) Representative: **Kador & Partner PartG mbB**
**Corneliusstraße 15**
**80469 München (DE)**

(54) **WAIST SUPPORT BELT**

(57)    In order to provide an industrially washable waist support belt having both industrial washing durability and stretchability, the waist support belt of the present invention includes a main body portion having a band shape, wherein the main body portion includes a base cloth, a male member of a hook-and-loop fastener, and a female member of a hook-and-loop fastener, the male member of the hook-and-loop fastener is sewn to the base cloth at a first end portion of a first surface of the main body portion, the female member of the hook-and-loop fastener is sewn to the base cloth at both end portions of a second surface of the main body portion, the male member of the hook-and-loop fastener contains a polyester elastomer resin in an amount of 95% by mass or more with respect to a mass of the male member of the hook-and-loop fastener, the female member of the hook-and-loop fastener contains one or more resins selected from the group consisting of a polyester resin and a polyamide resin in an amount of 95% by mass or more with respect to a mass of the female member of the hook-and-loop fastener, the base cloth is formed of one or more fibers selected from the group consisting of a polyester crimped fiber and a polyurethane elastic fiber, and a first index of the base cloth [stress at 25% elongation (N/50 mm) of base cloth/hysteresis loss rate (%)] $\times$ [shearing stress (N) of waist support belt] is 60 to 300.

Fig. 3

EP 4 104 703 A1

**Description**

TECHNICAL FIELD

The present invention relates to a waist support belt.

BACKGROUND ART

Among occupational diseases, low back pain is one of diseases incident to workers during working, and is spreading in various types of occupations. Examples of occupational workers who have a high risk of being attacked by low back pain include transportation operators who are engaged in handling heavy weight articles and in long-distance driving and medical workers working for assistance of inpatients and treating emergency patients, and recently, the number of workers relating to services in social welfare facilities for care of the elderly and the like and suffering from low back pain has considerably increased.

The number of persons suffering from low back pain tends to increase as the age of the professional workers increases. However, only a few people take a measure at an initial stage of low back pain, and there are not a few people who take no step until the low back pain gets worse. In addition, Japan is facing a problem with a decreased working-age population due to low birthrate and longevity, and there is an increasing number of senior workers of 65 years old or older and woman workers in the light of support of a society. Such being the case, importance of maintenance and promotion of health of valuable workers is increasing more than before. Also in case of working uniforms, a viewpoint of keeping health of workers positively becomes important in addition to conventional functions such as safety, mobility and discrimination.

Therefore, a waist support belt and a waist supporter that reduce a burden on the waist by tightening and fixing the pelvis have been generally known (refer to Patent Documents 1 and 2). The waist support belt is a waist support belt including a belt main body portion that can be wound around the waist and fastened to reinforce a pelvis, a right operation belt in which one is fixed to substantially the center of a belt main body and the other is configured to be detachable from an end of the belt main body, and a left operation belt in which one is fixed to substantially the center of the belt main body and the other is configured to be detachable from an end of the belt main body. In addition, the waist supporter is a supporter for a waist in which a supporter main body having a wide waist protection zone at the center and a fastening zone that can be fastened on both sides thereof is provided, an auxiliary band narrower than the supporter main body is provided on one side surface of the supporter main body, and an insertion hole is formed so that the auxiliary band can be pulled out from one side surface of the supporter main body to the other side surface. Further, Patent Document 2 discloses that a supporter main body of a waist supporter includes an elastic base material having shrinkability, such as Neoprene sponge, sponge rubber, or polyurethane foam, and includes an elastic base material having nylon on both side surfaces thereof, and a covering cloth knitted and woven with nylon fiber or another appropriate fiber material is integrally bonded to both side surfaces thereof, and the covering material on one side surface side has a surface formed in a loop such that a hook-and-loop fastener is locked, and a thread stitching or the like is provided on a peripheral edge thereof.

PRIOR ART DOCUMENTS

PATENT DOCUMENTS

Patent Document 1: Japanese Patent Laid-open Publication No. 2017-179663
Patent Document 2: Japanese Patent Laid-open Publication No. H8-182794

SUMMARY OF THE INVENTION

PROBLEMS TO BE SOLVED BY THE INVENTION

Incidentally, in the waist support belt in the related art, there is generally a risk that a male member of a hook-and-loop fastener damages other clothes, and a material susceptible to heat having high stretchability is often used. Therefore, as hand washing is recommended, the waist support belt has a configuration that cannot withstand not only industrial washing but also washing in a household washing machine.

For example, when the waist support belt as disclosed in Patent Document 1 or Patent Document 2 is industrially washed, the waist support belt contracts, and each of the members contract, so that appearance defects (puckering) occur. That is, in industrial washing, usually, a physical, thermal, or chemical force, or a combination of these forces acts on an object to be washed. As a result, members constituting the waist support belt contract, and shrinkages and changes in appearance occur. Therefore, the hand washing is usually recommended for the waist support belt as described above.

On the other hand, since it takes much time and effort to hand wash the waist support belt, there is a demand for automatic cleaning in a washing machine, an industrial washing facility, or the like.

Therefore, the present inventors have devised an improvement to a waist support belt having industrial washing durability in which shrinkages and changes in appearance are suppressed to be small even when the waist support belt is automatically cleaned in the industrial washing facility or the like.

Here, the waist support belt having the industrial washing durability refers to a waist support belt having no significant shrinkage or deformation in appearance even when the industrial washing is performed. In order to exhibit the performance of the waist support belt, the stretchability is required. Furthermore, the performance of the waist support belt refers to reduction of a burden on the waist of the wearer. In addition, the stretchability refers to stretch-resilience property which is a force for recovering to the original dimension of the stretched waist support belt. As a countermeasure for improving the industrial washing durability of the waist support belt, it is possible to use a material having excellent dimensional stability. However, a material excellent in the dimensional stability generally has no stretchability, and a material excellent in the stretchability generally has poor dimensional stability. Therefore, when a material specialized in the stretchability is frequently used, significant shrinkage or deformation in appearance occurs when the waist support belt is washed.

Therefore, an object of the present invention is to provide a waist support belt having both industrial washing durability and stretchability (also referred to as stretch-resilience property).

SOLUTIONS TO THE PROBLEMS

For solving the above-described problems, the present invention discloses the following constitution and more preferable aspects.

(1) A waist support belt including a main body portion having a band shape, wherein the main body portion includes a base cloth, a male member of a hook-and-loop fastener, and a female member of a hook-and-loop fastener, the male member of the hook-and-loop fastener is sewn to the base cloth at a first end portion of a first surface of the main body portion, the female member of the hook-and-loop fastener is sewn to the base cloth at both end portions of a second surface of the main body portion, the male member of the hook-and-loop fastener contains a polyester elastomer resin in an amount of 95% by mass or more with respect to a mass of the male member of the hook-and-loop fastener, the female member of the hook-and-loop fastener contains one or more resins selected from the group consisting of a polyester resin and a polyamide resin in an amount of 95% by mass or more with respect to a mass of the female member of the hook-and-loop fastener, the base cloth is formed of one or more fibers selected from the group consisting of a polyester crimped fiber and a polyurethane elastic fiber, and a first index of the base cloth [stress at 25% elongation (N/50 mm) of base cloth/hysteresis loss rate (%)] × [shearing stress (N) of waist support belt] is 60 to 300.

(2) The waist support belt according to (1), wherein the male member of the hook-and-loop fastener is exposed on an entire surface between a position at 0.05 to 0.2 from the first end portion of the first surface of the main body portion when a length, the first surface of the main body portion, in a longitudinal direction of the main body portion is set to 1 and the first end portion of the first surface of the main body portion, the female member of the hook-and-loop fastener is exposed on an entire surface between a position at 0.2 to 0.3 from a first end portion of the second surface of the main body portion when the length, the second surface of the main body portion, in the longitudinal direction of the main body portion is set to 1 and the first end portion of the second surface of the main body portion, and the female member of the hook-and-loop fastener is exposed on the entire surface between a position of 0.2 to 0.3 from a second end portion of the second surface of the main body portion and the second end portion of the second surface of the main body portion when the length, the second surface of the main body portion, in the longitudinal direction of the main body portion is set to 1, and the second end portion of the second surface of the main body portion.

(3) The waist support belt according to (1) or (2), further including an auxiliary portion having a band shape, wherein the male member of the hook-and-loop fastener is located at both end portions of one surface of the auxiliary portion, a central portion of the auxiliary portion in a longitudinal direction of the auxiliary portion is sewn to the central portion of the main body portion in the longitudinal direction of the main body portion, and a second index of the auxiliary portion [stress at 25% elongation (N/50 mm) of the auxiliary portion × length (cm) of the main body]/[length (cm) of the auxiliary portion × hysteresis loss rate (%)] is 2.5 to 4.0.

(4) The waist support belt according to any one of (1) to (3), wherein the base cloth is a knitted fabric.

(5) The waist support belt according to any one of (1) to (4), wherein the base cloth is formed of a polyester crimped fiber.

(6) The waist support belt according to any one of (1) to (5), wherein the polyester crimped fiber is a bimetal composite fiber in which two types of polyester polymers having a difference in intrinsic viscosity of 0.30 or more are bonded to each other along a fiber length direction.

(7) The waist support belt according to any one of (1) to (6), wherein the polyester crimped fiber is a bimetal composite fiber in which two types of polyester polymers having a difference in intrinsic viscosity of 0.30 or more are bonded to each other in a side-by-side type along a fiber length direction.

(8) The waist support belt according to (6) or (7), wherein the two types of polyester polymers are two types of polyester polymers selected from polyethylene terephthalate, polytrimethylene terephthalate, and polybutylene terephthalate.

(9) The waist support belt according to any one of (1) to (8), wherein the polyester crimped fiber is a side-by-side type composite fiber formed of polytrimethylene terephthalate and polyethylene terephthalate.

(10) The waist support belt according to any one of (1) to (9), wherein the polyester crimped fiber has a fineness of 100 to 400 dtex.

EFFECTS OF THE INVENTION

According to the present invention, it is possible to provide a waist support belt having both industrial washing durability and stretchability.

BRIEF DESCRIPTION OF THE DRAWINGS

Fig. 1 is a view of a waist support belt of one embodiment of the present invention when a first surface side of a main body portion is viewed from a front.
Fig. 2 is a view of the waist support belt of one embodiment of the present invention in which one end portion of an auxiliary portion is removed from the main body portion when a second surface side of the main body portion is viewed from the front.
Fig. 3 is a view in which both end portions of the auxiliary portion are attached to the main body portion when the waist support belt of one embodiment of the present invention is viewed from the second surface side of the main body portion.
Fig. 4 is a sectional view taken along line A-A' of the waist support belt illustrated in Fig. 1.

EMBODIMENTS OF THE INVENTION

Hereinafter, the present invention will be described with reference to the drawings, but the present invention is not limited to the following disclosure.
Here, a waist support belt of one embodiment will be described.

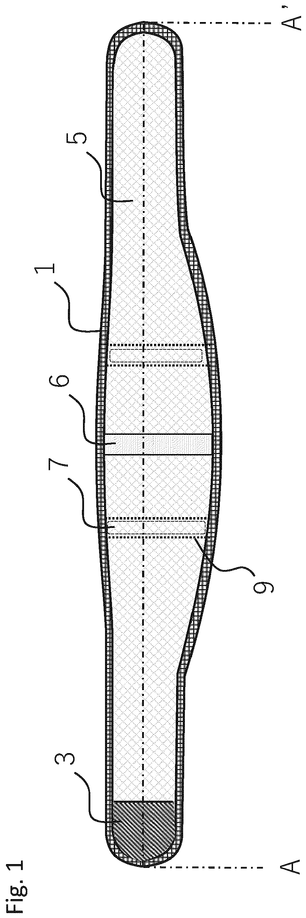

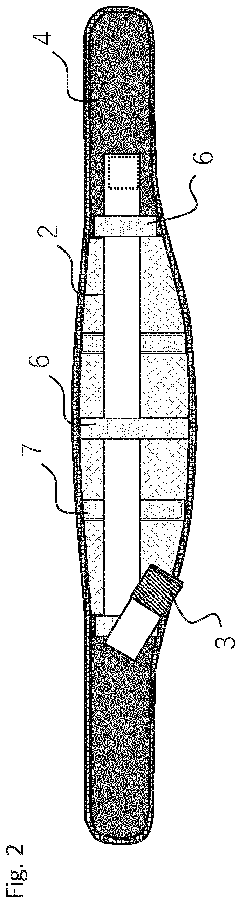

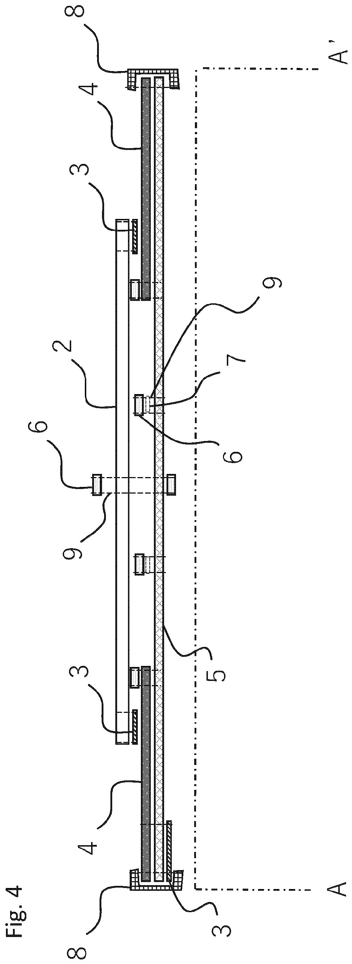

Fig. 1 is a view illustrating an example of an embodiment of a waist support belt of the present invention when a first surface side of a main body portion is viewed from a front. Fig. 2 is a view of the waist support belt illustrated in Fig. 1 when a second surface side of the main body portion is viewed from the front. In addition, Fig. 4 is a sectional view taken along line A-A' of the waist support belt illustrated in Fig. 1. The waist support belt of the present invention includes a main body portion 1 having a band shape and an auxiliary portion 2 having a band shape. By providing the auxiliary portion having the band shape in addition to the main body portion having the band shape as described above, a fastening pressure can be easily adjusted, and for example, the waist of the wearer can be protected with a necessary fastening pressure by a work load.
Here, the main body portion includes a base cloth 5, a male member 3 of a hook-and-loop fastener, and a female member 4 of a hook-and-loop fastener. Further, the male member of the hook-and-loop fastener is sewn to the base cloth at a first end portion of the first surface of the main body portion, and the female member of the hook-and-loop fastener is sewn to the base cloth at both end portions of the second surface of the main body portion. With the above configuration, the male member of the hook-and-loop fastener disposed at the first end portion of the first surface of the main body portion and the female member of the hook-and-loop fastener disposed at one end portion, not having the male member of the hook-and-loop fastener, of both end portions of the second surface of the main body portion form a locking portion, and the waist of the wearer can be fastened by the waist support belt.

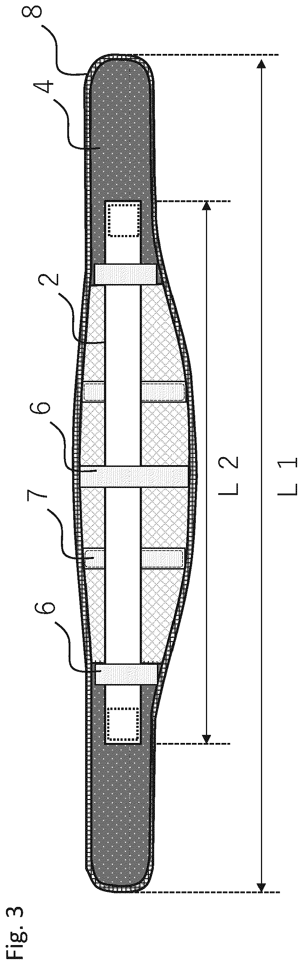

Fig. 3 is a view in which both end portions of the auxiliary portion are attached to the main body portion when the waist support belt of one embodiment of the present invention is viewed from the second surface side of the main body portion. Here, the male member of the hook-and-loop fastener is exposed on an entire surface between a position at 0.05 to 0.2 from the first end portion of the first surface of the main body portion when a length (L1 of Fig. 3), the first surface of the main body portion, in a longitudinal direction of the main body portion is set to 1 and the first end portion of the first surface of the main body portion, a female member of the hook-and-loop fastener is exposed on an entire surface between a position at 0.2 to 0.3 from a first end portion of the second surface of the main body portion when the length (L1 of Fig. 3), the second surface of the main body portion, in the longitudinal direction of the main body portion is set to 1 and the first end portion of the second surface of the main body portion, and the female member of the hook-and-loop fastener is exposed on the entire surface between a position of 0.2 to 0.3 from a second end portion of the second surface of the main body portion and the second end portion of the second surface of the main body portion when the length (L1 of Fig. 3), the second surface of the main body portion, in the longitudinal direction of the main body portion is set to 1, and the second end portion of the second surface of the main body portion. With the above configuration, an exposed area of the male member of the hook-and-loop fastener is smaller than the exposed area of the female member of the hook-and-loop fastener, and the fastening pressure of the main body portion can be adjusted depending on the attachment position of the male member of the hook-and-loop fastener to the female member of the hook-and-loop fastener, which is preferable. Furthermore, as d escribed above, when the male member of the hook-and-loop fastener disposed at the first end portion of the first surface of the main body portion and the female member of the hook-and-loop fastener disposed at one end portion, not having the male member of the hook-and-loop fastener, of both end portions of the second surface of the main body portion form a locking portion, it is possible to bring about a state in which the male member of the hook-and-loop fastener is not exposed at all. Accordingly, it is possible to prevent a male surface projection part of the male member of the hook-and-loop fastener from damaging other clothes when washing the waist support belt of the present invention.

In addition, since the larger the bonding area between the male member of the hook-and-loop fastener and the female member of the hook-and-loop fastener, the more firmly the connection is established, and the more sufficient the shearing stress is, it is preferable that as for the male member of the hook-and-loop fastener, the male member of the hook-and-loop fastener is exposed on the entire surface between a position at 0.07 to 0.2 from the first end portion of the first surface of the main body portion when a length (L1 of Fig. 3) in a longitudinal direction of the main body portion is set to 1 and the first end portion of the first surface of the main body portion, and it is more preferable that as for the male member of the hook-and-loop fastener, the male member of the hook-and-loop fastener is exposed on the entire surface between a position at 0.1 to 0.2 from the first end portion of the first surface of the main body portion when the length (L1 of Fig. 3) in the longitudinal direction of the main body portion is set to 1 and the first end portion of the first surface of the main body portion.

Here, the length of the main body portion in the longitudinal direction (L1 in Fig. 3) is preferably 1.07 to 1.60 times the waist circumference of the wearer. Commercial product design may be sized for each range of waist circumference. In this case, for example, when developing in three stages such as a waist circumference of 65 to 85 cm, 85 to 100 cm, and 100 to 110 cm, it is preferable to set the waist circumference to 100 cm when the waist circumference is 65 to 85 cm, 110 cm when the waist circumference is 85 to 100 cm, and 120 cm when the waist circumference is 100 to 110 cm. Here, the waist circumference refers to a waist size, and indicates a circumferential length in the middle position between the lowermost end of the rib and the hipbone. By setting the length of the main body portion in the longitudinal direction to 1.07 times or more of the waist circumference, a sufficient overlapping portion can be formed and the main body can be fastened without difficulty when the wearer wears the waist support belt. In addition, by setting the length of the main body portion in the longitudinal direction to 1.60 times or less the waist circumference, the overlapping portion when the wearer wears the support belt becomes appropriate, and it is possible to fasten the support belt with an appropriate pressure. From the above, the length of the main body portion in the longitudinal direction is preferably 1.07 to 1.60 times the waist circumference of the wearer. It is more preferable that the length is upper limit of the size expansion $\times$ 1.07 times or more, and the female member can be mounted in a state of covering the entire exposed surface of the male member when the wearer of the corresponding size wears. For the above reason, assuming a range of about 55 to 135 cm as a waist circumference of a wearer of a general physique, the length (L1 in Fig. 3) of the main body portion in the longitudinal direction is preferably about 75 to 150 cm in terms of ease of wearing. In addition, assuming a range of about 65 to 115 cm as a waist circumference frequently set commercially, the length of the main body portion in the longitudinal direction is preferably about 90 to 130 cm. By setting within such a range, an appropriate overlapping portion is generally formed when the wearer wears the waist support belt, and the waist support belt can be fastened without difficulty.

In addition, the male member of the hook-and-loop fastener contains a polyester elastomer resin in an amount of 95% by mass or more with respect to the mass of the male member of the hook-and-loop fastener. The polyester elastomer resin is generally excellent in heat resistance. Therefore, by using a polyester elastomer resin in an amount of 95% by mass or more with respect to the mass of the male member of the hook-and-loop fastener, it is possible to contribute to suppressing shrinkage and deformation in appearance of the member in a step to which high temperature heat is applied, such as a washing step and a drying step during industrial washing. Therefore, the male member of the hook-and-loop fastener contains the polyester elastomer resin in an amount of 95% by mass or more with respect to the mass of the male member of the hook-and-loop fastener.

In addition, the male member of the hook-and-loop fastener may contain components other than the polyester elastomer resin, and for example, may contain an assistant such as a pigment or a flame retardant.

The type of the male member of the hook-and-loop fastener is not particularly limited. However, the male member of the hook-and-loop fastener is likely to cause pilling on the surface of other clothes when the male surface projection damages other clothes. In consideration of this, it is preferable to use a molded body in the form of a "mushroom type" in which a tip end portion of the male surface projection is rounded.

Examples of the polyester elastomer include a thermoplastic rubber elastic body having a hard segment having a polyester-based structure such as polyethylene terephthalate (PET), polytrimethylene terephthalate (PTT), and polybutylene terephthalate (PBT) and a soft segment. The type of the polyester resin is not particularly limited, but a block copolymer of a hard segment of polybutylene terephthalate and a soft segment of polyether is preferable from the viewpoint of excellent heat resistance and durability.

Further, the female member of the hook-and-loop fastener contains at least one resin selected from the group consisting of a polyester resin and a polyamide resin in an amount of 95% by mass or more with respect to the mass of the female member of the hook-and-loop fastener.

Here, the polyester resin is not particularly limited, and examples thereof include polyalkylene terephthalate resins such as a polyethylene terephthalate resin, a polytrimethylene terephthalate resin, and a polybutylene terephthalate resin.

The polyamide resin is not particularly limited, and examples thereof include a nylon resin and an aramid resin. Here, the nylon resin refers to a polyamide containing an aliphatic skeleton such as nylon 6 and nylon 66, and the aramid resin refers to an aromatic polyamide such as polyparaphenylene terephthalamide, metaphenyleneisophthalamide, or a copolymer having these as a main skeleton.

Although the polyamide resin is slightly inferior in dimensional stability of the polyester resin, both the polyester resin and the polyamide resin are resins having sufficient dimensional stability. By employing the polyamide resin for the material of the female member of the hook-and-loop fastener, it is possible to sufficiently contribute to the suppression of shrinkage and deformation in appearance of the member in a step to which high temperature heat is applied, such as a washing step and a drying step during the industrial washing. Furthermore, the polyamide resin is generally excellent in wear resistant, and by using the polyamide resin for the female member of the hook-and-loop fastener, it is possible to suppress deterioration when the hook-and-loop fastener is repeatedly attached and detached or when friction with other clothes occurs in the washing step during the industrial washing.

In addition, the molded body of the polyester resin is particularly excellent in dimensional stability, and by employing the polyester resin as the material of the female member of the hook-and-loop fastener, it is possible to greatly contribute to the suppression of shrinkage and deformation in appearance of the member in a step to which high temperature heat is applied, such as a washing step and a drying step during the industrial washing.

Here, as long as the female member of the hook-and-loop fastener contains at least one resin selected from the group consisting of a polyester resin and a polyamide resin in an amount of 95% by mass or more with respect to the mass of the female member of the hook-and-loop fastener, components other than the polyester resin and the polyamide resin may be contained, and for example, an assistant such as a pigment or a flame retardant may be contained. In order to produce a waist support belt having more excellent dimensional stability, it is more preferable that the female member of the hook-and-loop fastener contains the polyester resin in an amount of 95% by mass or more with respect to the mass of the female member of the hook-and-loop fastener.

By using the female member of the hook-and-loop fastener as described above, it is possible to suppress shrinkage and change in appearance of the member during the washing step or the drying step during the industrial washing, and/or improve durability against friction with other clothes in the washing step when the waist support belt is repeatedly used.

Examples of the female member of the hook-and-loop fastener include those made of a knitted fabric or a woven fabric. Examples of the knitted fabric include a circular knitted fabric, a weft knitted fabric, and a warp knitted fabric, but are not particularly limited. Examples of the knitted fabric used for the female member of the hook-and-loop fastener include a knitted fabric having a plain surface and a knitted fabric having a mesh surface, and a knitted fabric having a mesh surface is more preferable. The fabric having a mesh surface has a smaller volume and a smaller amount of yarns than the fabric having no mesh surface of the same size and the same material. If the yarn amount is small, the absolute value of the dimensional change also becomes small. Therefore, in order to suppress the dimensional change of the female member of the hook-and-loop fastener to be small, it is more preferable to use the female member of the mesh-like hook-and-loop fastener. In addition, as the woven fabric used for the female member of the hook-and-loop fastener, a woven fabric having a looped surface is preferable. In general, since the woven fabric is superior in the dimensional stability to the knitted fabric, when used as the hook-and-loop fastener female member of the waist support belt, the dimensional change rate during washing can be further reduced. Therefore, in order to obtain a waist support belt having more excellent dimensional stability, the female member of the hook-and-loop fastener is more preferably made of a woven fabric having a looped surface.

The base cloth is formed of one or more fibers selected from the group consisting of polyester crimped fibers and polyurethane elastic fibers. Here, the crimped fiber generally refers to a composite fiber formed of a high melting point component and a low melting point component having different melting points from each other, a composite fiber formed of a high viscosity component and a low viscosity component having different intrinsic viscosities from each other, or a composite fiber formed of a high shrinkage component and a low shrinkage component having different shrinkage rates, and is a fiber in which three-dimensional spiral crimps become apparent when one component such as the low melting point component and the high shrinkage component is more strongly activated by heat than the other component, and the fiber has stretchability and elasticity due to the three-dimensional spiral crimps. Here, the crimped fiber is a polyester crimped fiber. As described above, the polyester resin has excellent dimensional stability even when exposed to high temperature heat in the washing and the drying step during the industrial washing, and thus can suppress shrinkage of the cloth and the member.

As described above, in the polyester crimped fiber, crimps become apparent by heat to form a crimped fiber. The appearance of crimps may be performed at a stage before the base cloth is formed, that is, in the form of fibers, or after the base cloth is formed. In the present invention, a raw yarn before the appearance of crimps is also referred to as a crimped fiber for convenience.

In particular, in order to obtain a polyester crimped fiber having excellent stretchability, the high viscosity component (high shrinkage component) and the low viscosity component (low shrinkage component) of the polyester crimped fiber are preferably a bimetallic composite fiber in which two types of polyester polymers having a difference in intrinsic viscosity of 0.30 or more are bonded in a side-by-side type along the fiber length direction. By combining these, a raw yarn having excellent crimp characteristics is obtained. In particular, when a difference in intrinsic viscosity is increased to 0.50 or more, a raw yarn further excellent in the stretchability is obtained, which is preferable. On the other hand, when the difference in intrinsic viscosity is more than 1.50, the crimping characteristics of the obtained yarn are good, but the spun yarn is excessively bent toward the high viscosity component, so that the yarn cannot be stably produced for a long time, which is not preferable. Therefore, in order to satisfy both stable yarn making properties and stretch recovery properties, a difference in intrinsic viscosity is desirably 0.30 or more and 1.50 or less.

The two types of polyester polymers are preferably two types of polyester polymers selected from polyethylene terephthalate, polytrimethylene terephthalate, and polybutylene terephthalate. Here, the combination of the polyester polymers is not particularly limited, and when two types of polyethylene terephthalates having a difference in intrinsic viscosity are used, a certain degree of stretchability can be obtained, but there is a problem that the stretchability in a woven fabric is insufficient, and it is difficult to obtain a satisfactory stretchable woven fabric. Therefore, a combination of polyethylene terephthalate/polytrimethylene terephthalate, polyethylene terephthalate/polybutylene terephthalate, or polytrimethylene terephthalate/polybutylene terephthalate is preferable.

The stretch-resilience property is determined by a diameter of the three-dimensional spiral crimp and the number of crimps of the single filament. The diameter of the three-dimensional spiral crimp and the number of crimps of the single filament may be determined by a difference in shrinkage (including a difference in elastic recovery rate) between the high melting point component and the low melting point component caused by a difference in intrinsic viscosity. The larger the difference in shrinkage, the smaller the diameter of the spiral crimp and the larger the number of crimps per unit filament. As a stretch material, it is preferable that the diameter of the spiral crimp is small, the number of crimps per unit filament is large (that is, it is excellent in stretchability and good in appearance), and permanent set-in fatigue resistance of the spiral crimp is good.

From the above reasons, as the polyester crimped fiber having excellent stretchability and excellent appearance, a combination in which at least one component of a polymer is polytrimethylene terephthalate, such as polyethylene terephthalate/polytrimethylene terephthalate having a relatively large shrinkage difference, or a combination of polyethylene terephthalate/polybutylene terephthalate can be more preferably exemplified.

Further, a polyester crimped fiber in which at least one component of the polymer is polytrimethylene terephthalate is more preferable. This is because polytrimethylene terephthalate has mechanical characteristics and chemical characteristics equivalent to those of polyethylene terephthalate and polybutylene terephthalate, which are representative polyester long fibers, and is extremely excellent in the stretch-resilience property.

Furthermore, since the stretchability of the high melting point component (high shrinkage component) with the low melting point component (low shrinkage component) as a fulcrum is dominant in the stretchability of the spiral crimp, the polymer used for the high melting point component preferably has high stretchability and recoverability. Specifically, it is preferable to employ polyethylene terephthalate as the low melting point component and polytrimethylene terephthalate as the high melting point component.

The single fiber cross-sectional shape of the polyester crimped fiber used in the present invention is preferably a side-by-side type or an eccentric sheath-core type. Since the cross-sectional shape is a side-by-side type or an eccentric sheath-core type, spiral crimps are developed when heat is applied to the yarn, and stretchability can be imparted to the yarn. Among them, the single fiber cross-sectional shape of the polyester crimped fiber used in the present invention is more preferably a side-by-side type because the side-by-side type structure is simpler and more excellent in spinnability. From the above, the polyester crimped fiber is preferably a bimetal composite fiber in which two types of polyester polymers having a difference in intrinsic viscosity of 0.30 or more are bonded in a side-by-side type along the length direction.

In addition, as the resin type, the two types of polyester polymers are preferably two types of polyester polymers selected from polyethylene terephthalate, polytrimethylene terephthalate, and polybutylene terephthalate. Among them, a side-by-side bimetal composite fiber formed of polytrimethylene terephthalate and polyethylene terephthalate is most preferable.

The polyurethane elastic fiber is a fiber having a urethane bond, having a structure in which a flexible "soft" segment and a hard "hard" segment are bonded, and having continuous and very high stretchability. In addition, it is known that polyurethane elastic fibers have high dimensional stability by undergoing a heat setting step. Therefore, when polyurethane elastic fibers are employed for the base cloth, the sufficient dimensional stability can be imparted to the base cloth by performing heat setting at the time of producing the base cloth.

As described above, both the polyester crimped fibers and the polyurethane elastic fibers are elastic fibers having the stretchability and the elasticity, and when a base cloth is produced using these elastic fibers, the stretchability and the elasticity are also developed in the base cloth. Due to this stretchability and elasticity, when the base cloth is stretched left and right or up and down, a stretch recovery force to return the base cloth to the original shape is generated. By utilizing this phenomenon, when the base cloth is fixed by the above-described locking portion and wound around the waist in a state of being slightly stretched, the waist can be fastened with a constant pressure.

For the above reason, the base cloth is formed of one or more fibers selected from the group consisting of a polyester crimped fiber and a polyurethane elastic fiber. Here, only one of the polyester crimped fiber and the polyurethane elastic fiber may be used, or both of them may be used in combination. Fibers other than the polyester crimped fiber and the polyurethane elastic fiber may be used, and in that case, the polyester crimped fiber and/or the polyurethane elastic fiber are preferably contained in an amount of 20% by mass or more with respect to the mass of the base cloth. When the polyester crimped fiber and/or the polyurethane elastic fiber are contained in an amount of 20% by mass or more with respect to the total mass of the base cloth, the stretchability and elasticity of the base cloth are further improved.

When only the polyester crimped fiber is used for the base cloth, the upper limit of the amount used is not particularly limited, and the polyester crimped fiber may be 100% by mass. On the other hand, when the polyurethane elastic fiber is used, the upper limit of the content of the polyurethane elastic fiber is preferably 80% by mass. In general, the polyurethane elastic fiber is broken when excessive tension is applied. Therefore, when the base cloth is formed of 20% by mass or more of fibers other than the polyurethane elastic fiber, sufficient strength is provided to the base cloth, which is preferable. From the above reasons, when the polyurethane elastic fiber is used, the content of the polyurethane elastic fiber is preferably 80% by mass or less.

Furthermore, the fineness of the polyester crimped fiber is preferably 100 to 400 dtex, and the fineness of the polyurethane elastic fiber is preferably 1000 to 1500 dtex.

When the fineness of the polyester crimped fiber is 100 dtex or more, the stress at elongation and stretch-resilience property of the base cloth used for the main body portion become sufficient, and when the waist support belt employing the base cloth is fixed by the locking portion in a slightly stretched state and wound around the waist, the waist can be fastened with sufficient pressure. When the fineness of the polyester crimped fiber is 400 dtex or less, appropriate rigidity and adhesion to the body can be obtained. Furthermore, by setting the fineness of the polyester crimped fiber to 200 dtex or less, the stress at elongation and the stress at recovery to be described later of the base cloth used for the main body portion are not excessively high, and sufficient stretchability can be imparted.

By setting the fineness of the polyurethane elastic fiber to 1000 dtex or more, the stress at elongation and the stretch-resilience property of the base cloth used for the main body portion become sufficient, and when the waist support belt employing the base cloth is fixed by the locking portion in a slightly stretched state and wound around the waist, the waist can be fastened with sufficient pressure. In addition, by setting the fineness of the polyurethane elastic fiber to 1500 dtex or less, the stress at elongation and the stress at recovery to be described later of the base cloth used for the main body portion are not excessively high, and the process passability at the time of knitting can also be excellently maintained.

From the above reasons, the fineness of the polyester crimped fiber is preferably 100 to 400 dtex, and the fineness of the polyurethane elastic fiber is preferably 1000 to 1500 dtex.

The base cloth is preferably a knitted fabric. As described above, the base cloth used for the industrial washable waist support belt is required to have the dimensional stability and the stretchability. Regarding the dimensional stability, the required dimensional stability can be obtained by using the above-mentioned material for the base cloth. On the other hand, the stretchability is exhibited only by the characteristics of the above-described material, but the base cloth is preferably a knitted fabric in order to obtain a waist support belt that fits the wearer's body. Since the base cloth is a knitted fabric, the structural stretchability of the base cloth is also added, and a waist support belt having more excellent stretchability is obtained. The type of knitted fabric is not particularly limited, and examples thereof include a weft knitted fabric, a circular knitted fabric, and a warp knitted fabric. Among them, the warp knitted fabric is more preferable from the viewpoint that the direction of expansion and contraction of the base cloth can be relatively freely designed.

Here, the first index of the base cloth [stress at 25% elongation (N/50 mm) of base cloth/hysteresis loss rate (%)] × [shearing stress (N) of waist support belt] is 60 to 300.

Here, the stress at 25% elongation of the base cloth is a value obtained by sampling a test piece so that the direction parallel to the longitudinal direction of the main body portion of the waist support belt is the longitudinal direction of the base cloth, and by measuring the stress at 25% elongation described later. When this value is large, it indicates that the base cloth is difficult to stretch and the force applied to stretch the base cloth is large. On the other hand, when this value is small, it indicates that the base cloth is easy to stretch and the force applied to stretch the base cloth is small.

Therefore, the stress at 25% elongation of the base cloth is preferably 5 N/50 mm or more and 35 N/50 mm or less. When the stress at 25% elongation of the base cloth is 5 N/50 mm or more, the waist support belt elongation force can be further improved. In addition, when the stress at 25% elongation of the base cloth is 35 N/50 mm or less, the elongation force of the base cloth does not become excessively high, and the wearing feeling is excellent. Here, the smaller the stress at 25% elongation is, the more easily the cloth stretches and the better the fitting when worn, and therefore, the stress is more preferably 25 N/50 mm or less.

The hysteresis loss rate indicates the degree of strain that can be calculated from the stress at elongation and the stress at recovery when the base cloth is made to have the original length at the same speed after the stress at 25% elongation of the base cloth is measured. The hysteresis loss rate can be calculated by multiplying a value obtained by subtracting an integral value of stress (stress at recovery) from 0% elongation to 25% elongation from an integral value of stress (stress at elongation) from 25% elongation to 0% elongation by the integral values of stress (stress at elongation) from 0% elongation to 25% elongation by 100. When the hysteresis loss rate of the base cloth is large, it indicates that the base cloth is easily fatigued. On the other hand, when the hysteresis loss rate of the base cloth is small, it indicates that the base cloth is hardly fatigued and is excellent in stretch-resilience property (stretchability). From the above viewpoint, the hysteresis loss rate of the base cloth is preferably 20% or more and 70% or less. When the hysteresis loss rate of the base cloth is 20% or more, the cloth slightly stretches and fits when the waist support belt is worn, so that the fastening pressure is not excessively increased and the wearing feeling is good. In addition, since the hysteresis loss rate of the base cloth is 70% or less, the base cloth is not excessively fatigued, and the body of the wearer can be fastened with a constant pressure even when the waist support belt is repeatedly used. As described above, since the smaller the hysteresis loss rate, the better the stretch-resilience property, the hysteresis loss rate is more preferably 50% or less, and still more preferably 30% or less.

The shearing stress of the waist support belt is a value obtained by shearing stress measurement of the hook-and-loop fastener according to JIS L 3416 (2010) described later in a state where the female member of the hook-and-loop fastener of the waist support belt main body portion covers the entire exposed surface of the male member of the hook-and-loop fastener of the waist support belt main body portion. When the value of the shearing stress of the waist support belt is large, it indicates that the locking portion of the waist support belt is hardly detached and the body of the wearer is firmly fastened, and conversely, when the value of the shearing stress of the waist support belt is small, it indicates that the locking portion of the waist support belt is easily detached and the force for fastening the body of the wearer is reduced. From the above viewpoint, the shearing stress of the waist support belt is preferably 200 N or more and 400 N or less. When the shearing stress of the waist support belt is 200 N or more, the bonding strength of a waist support belt locking portion can be further improved. In addition, since the shearing stress of the waist support belt is 400 N or less, the wearer can perform the operation without applying force when removing the waist support belt, and the workability at the time of attaching and detaching the waist support belt can be excellently maintained.

From the above, it can be said that when the stress at 25% elongation (N/50 mm) of the base cloth is in the above range, a waist support belt excellent in the wearing feeling with an appropriate elongation force is obtained, when the hysteresis loss rate (%) is in the above range, the waist support belt is not fatigued and a constant fastening pressure can be maintained, and when the shearing stress (N) of the waist support belt is in the above range, the waist support belt is not detached, and the workability at the time of attaching and detaching the waist support belt can be excellently maintained. Therefore, the first index of the base cloth [stress at 25% elongation (N/50 mm) of base cloth/hysteresis loss rate (%)] × [shearing stress (N) of waist support belt] indicates whether or not the waist support belt is a waist support belt that can be worn for a long time without being detached while maintaining a constant tension with an appropriate tension.

Here, in order to secure the minimum performance of the waist support belt that reduces and protects the load on the wearer's body, the lower limit of the first index of the base cloth indicated using the above value is 60. In addition, if the value of the stress at 25% elongation of the base cloth is excessively large, the wearing feeling of the wearer is adversely affected, and if the value of the shearing stress of the waist support belt is excessively large, it is difficult to attach and detach the waist support belt locking portion, so that the upper limit of the first index of the base cloth is 300. For the above reasons, the first index of the base cloth is 60 to 300.

In addition, the waist support belt of the present invention preferably includes an auxiliary portion. As indicated by reference numeral 3 in Fig. 2, male members of the hook-and-loop fastener are located at both ends of one surface of the auxiliary portion. With such a configuration, both ends of the auxiliary portion can be attached to the female member of the hook-and-loop fastener disposed on the second surface of the main body portion of the waist support belt, and the elongation degree of the auxiliary portion is changed by adjusting the attachment position, and thereby the fastening pressure on the body of the wearer can be adjusted. Here, a central portion of the auxiliary portion in the longitudinal direction of the auxiliary portion is sewn to a central portion of the main body portion in the longitudinal direction of the main body portion. With such a configuration, a starting point when the auxiliary portion is stretched is formed at the central portion of the auxiliary portion in the longitudinal direction of the auxiliary portion. With this starting point, the auxiliary portion can be stretched with the central portion of the waist support belt as a fulcrum and attached to the female member of the hook-and-loop fastener, and the body of the wearer can be more firmly fastened.

Here, a second index of the auxiliary portion [stress at 25% elongation (N/50 mm) of the auxiliary portion × length (cm) of the main body portion]/[length (cm) of the auxiliary portion × hysteresis loss rate (%)] is preferably 2.5 to 4.0. Here, the second index of the auxiliary portion refers to a value obtained by further dividing a value obtained by dividing a stress (N/50 mm) at 25% elongation of the auxiliary portion by a ratio (length (cm) of the auxiliary portion/length (cm) of the main body portion) of a length (cm) of the auxiliary portion and a length (cm) of the main body portion by a hysteresis loss rate (%).

Here, the stress at 25% elongation of the auxiliary portion is a value obtained by sampling a test piece so that the direction parallel to the longitudinal direction of the main body portion of the waist support belt is the longitudinal direction of the auxiliary portion, and by measuring the stress at 25% elongation described later. When this value is large, it indicates that the auxiliary portion is difficult to stretch and the power is large. On the other hand, when this value is small, it indicates that the auxiliary portion is easy to stretch and the power is small. From the above viewpoint, the stress at 25% elongation of the auxiliary portion is preferably 10 N/50 mm or more and 30 N/50 mm or less. When the stress at 25% elongation of the auxiliary portion is 10 N/50 mm or more, a minimum fastening pressure can be applied when the auxiliary portion is stretched and attached to the female member of the hook-and-loop fastener of the main body portion. Furthermore, since a sufficient fastening pressure can be applied, it is more preferably 15 N/50 mm or more. In addition, since the stress at 25% elongation of the auxiliary portion is 30 N/50 mm or less, when the auxiliary portion is stretched and attached to the female member of the hook-and-loop fastener of the main body portion, the wearer can easily attach the auxiliary portion without applying much force.

The length of the auxiliary portion is indicated by L2 in Fig. 3, and refers to the length of the auxiliary portion in a direction parallel to the longitudinal direction of the main body portion. The length of the main body portion is indicated by L1 in Fig. 3, and refers to the length of the main body portion in the longitudinal direction. The ratio of the length of the auxiliary portion to the length of the main body portion (length of auxiliary portion/length of main body portion) is preferably 0.45 or more and 0.65 or less. When the ratio of the length of the auxiliary portion to the length of the main body portion (length of auxiliary portion/length of main body portion) is 0.45 or more, the wearer easily stretches the auxiliary portion when stretching the auxiliary portion and attaches the auxiliary portion to the female member of the hook-and-loop fastener of the main body portion. In addition, when the ratio of the length of the auxiliary portion to the length of the main body portion (length of auxiliary portion/length of main body portion) is 0.65 or less, an adjustment region of the elongation to stretch the auxiliary portion can be secured when the wearer stretches the auxiliary portion and attaches the auxiliary portion to the female member of the hook-and-loop fastener of the main body portion, and the fastening pressure can be adjusted in accordance with the wearer's body shape and work load.

The hysteresis loss rate of the auxiliary portion indicates the degree of strain that can be calculated from the stress at elongation and the stress at recovery when the auxiliary portion is made to have the original length at the same speed after the stress at 25% elongation of the base cloth is measured. The hysteresis loss rate of the auxiliary portion can be calculated by multiplying a value obtained by subtracting an integral value of stress (stress at recovery) from 0% elongation to 25% elongation from an integral value of stress (stress at elongation) from 25% elongation to 0% elongation by a sum of integral values of stress (stress at elongation) from 0% elongation to 25% elongation by 100. When the hysteresis loss rate of the auxiliary portion is large, it indicates that the auxiliary portion is easily fatigued. On the other hand, when the hysteresis loss rate of the auxiliary portion is small, it indicates that the auxiliary portion is hardly fatigued and is excellent in stretch-resilience property.

From the above viewpoint, the hysteresis loss rate of the auxiliary portion is preferably 5% or more and 20% or less. When the hysteresis loss rate of the auxiliary portion is 5% or more, the fastening pressure does not excessively increase when the auxiliary portion is stretched and attached to the female member of the hook-and-loop fastener of the main body portion, and the wearing feeling is excellent.

In addition, since the hysteresis loss rate of the auxiliary portion is 20% or less, the auxiliary portion is not excessively fatigued, and the body of the wearer can be fastened with a constant pressure even when the waist support belt is repeatedly used. As described above, since the smaller the hysteresis loss rate, the better the stretch-resilience property, and the hysteresis loss rate is more preferably 15% or less.

From the above, it can be said that when the stress (N/50 mm) at 25% elongation of the auxiliary portion is in the above range, the waist support belt has an appropriate fastening pressure, and when the length (cm) of the auxiliary portion/the length (cm) of the main body portion is in the above range, the workability at the time of adjusting the fastening pressure according to the wearer's body shape and the work load is excellent, the adjustment range of the fastening pressure is also an optimum waist support belt, and when the hysteresis loss rate of the auxiliary portion is in the above range, the waist support belt can keep the appropriate fastening pressure constant. Therefore, the second index of the auxiliary portion [stress at 25% elongation (N/50 mm) of the auxiliary portion × length (cm) of the main body portion] /[length (cm) of the auxiliary portion × hysteresis loss rate (%)] is an appropriate fastening pressure, and adjustment of the fastening pressure can be performed within an optimum range, and indicates whether or not the waist support belt can keep the fastening pressure constant.

Here, in order to secure the minimum performance of the waist support belt that reduces and protects the load on the wearer's body, the second index of the auxiliary portion indicated using the above value is 2.5 or more. In addition, if the value of the stress at 25% elongation of the auxiliary portion is excessively large, it becomes difficult to attach the auxiliary portion while pulling the auxiliary portion when attaching the auxiliary portion to the female member of the hook-and-loop fastener disposed on the second surface of the main body portion, and even if the value of the ratio of the length of the auxiliary portion of the waist support belt to the length of the main body portion (length of auxiliary portion/length of main body portion) is excessively small, it becomes difficult to attach the auxiliary portion while pulling the auxiliary portion when attaching the auxiliary portion to the female member of the hook-and-loop fastener disposed on the second surface of the main body portion, so that the second index of the auxiliary portion is preferably 4.0 or less. From the above reason, the second index of the auxiliary portion is preferably 2.5 to 4.0.

In addition, the waist support belt of the present invention may include an auxiliary material in addition to the main body portion having a band shape and the auxiliary portion having a band shape. Here, examples of the auxiliary material include a bone as indicated by reference numeral 7 in Fig. 1, a tape as indicated by reference numeral 6 in Fig. 2, and a piping tape as indicated by reference numeral 8 in Fig. 3. Here, when the auxiliary material is used, the following materials are preferable in consideration of industrial washing durability.

As the bone, a resin plate made of polyethylene or polypropylene is preferably used from the viewpoint of excellent heat resistance and suppressed thermal deformation.

As the tape, it is preferable to form a material having excellent dimensional stability as a woven fabric having excellent dimensional stability, and specifically, it is preferable to use a woven fabric of 100% polyester fibers.

As the piping tape, a polyamide fiber excellent in the abrasion resistance or a polyester fiber excellent in the dimensional stability is preferable as the material to be used, and it is preferable to use at least one of the polyamide fiber and the polyester fiber as the material to be used in order to cover the periphery of the end portion of the waist support belt main body as illustrated in Fig. 4. Furthermore, since the waist support belt main body has stretchability, it is preferable that the piping tape also has stretchability so as to follow the stretching of the main body portion, and from this viewpoint, polyurethane elastic fibers are also preferably used. Furthermore, in order to maintain the dimensional stability during the industrial washing, the piping tape is preferably a woven fabric, and the piping tape is preferably a woven fabric using polyamide fibers or/and polyester fibers and polyurethane elastic fibers. Among them, in particular, since the dimensional change rate of the entire waist support belt can be reduced by using a polyester fiber excellent in the dimensional stability for the piping tape, it is more preferable to use a woven fabric of 100% polyester fiber.

Here, the disposition of the auxiliary material is not particularly limited, and as illustrated in Fig. 4, it is preferable that the auxiliary material is stacked on the first surface and the second surface of the base cloth, and sewn with a sewing machine or the like as shown by a perforation 9. However, since there is a possibility that the stretchability of the waist support belt main body portion is impaired due to the perforation, the number of parts of the auxiliary material is preferably suppressed to 10 or less.

Examples

Hereinafter, the present invention will be described in further detail by way of examples.

(Measuring Method)

(Stress at 25% elongation)

The stress at 25% elongation of a base cloth and an auxiliary portion sample was measured using Autograph AG-50kNG manufactured by Shimadzu Corporation. Specifically, five test pieces each having a width of 50 mm and a length of 220 mm were prepared. Next, one of the five test pieces was attached to the measuring instrument at a distance between chucks of 100 mm, and the test piece was pulled at a pulling speed of 300 mm/min up to a point at which the test piece stretched by 25%, and returned to the original length. The other four test pieces were subjected to the same treatment as described above, and the stress at 25% elongation of each of the five test pieces was measured. The "stress at 25% elongation" mentioned here is an average of the elongation stresses of the five fabrics at an elongation of 25% as measured by the above-described method. Here, when the stress at 25% elongation of the base cloth is measured, the test piece is sampled from the base cloth so that the length direction of the test piece is the same as the direction parallel to the longitudinal direction of the main body portion of the waist support belt, and when the stress at 25% elongation of the auxiliary portion is measured, the test piece is sampled from the auxiliary portion so that the length direction of the test piece is the same as the direction parallel to the longitudinal direction of the main body portion of the waist support belt.

(Hysteresis loss rate)

The stress at 25% elongation of a base cloth and an auxiliary portion sample was measured using Autograph AG-50kNG manufactured by Shimadzu Corporation. Specifically, five test pieces each having a width of 50 mm and a length of 220 mm were prepared. Next, one of the five test pieces was attached to the measuring instrument at a distance between chucks of 100 mm, and the test piece was pulled at a pulling speed of 300 mm/min up to a point at which the test piece stretched by 25%, and returned to the original length at the same speed. The same was performed for the other four test pieces, and for each of the five test pieces, the stress (stress at elongation) from 0% elongation to 25% elongation and the stress (stress at recovery) from 25% elongation to 0% elongation were measured over time every 0.2 seconds. For the measured values, the sum of the integrated values of the stresses (stresses at elongation) from 0% elongation to 25% elongation and the sum of the integrated values of the stresses (stresses at recovery) from 25% elongation to 0% elongation were calculated, the sum of the integrated values of the stresses (stresses at recovery) from 25% elongation to 0% elongation was subtracted from the sum of the integrated values of the stresses (stresses at elongation) from 0% elongation to 25% elongation, and the value divided by the sum of the integrated values of the stresses (stresses at elongation) from 0% elongation to 25% elongation was multiplied by 100 to calculate the hysteresis loss rate. The "hysteresis loss rate" as used herein refers to an average value of the hysteresis loss rates of five pieces of fabric measured by the above method. Here, when the hysteresis loss rate of the base cloth is measured, the test piece is sampled from the base cloth so that the length direction of the test piece is the same as the direction parallel to the longitudinal direction of the main body portion of the waist support belt, and when the hysteresis loss rate of the auxiliary portion is measured, the test piece is sampled from the auxiliary portion so that the length direction of the test piece is the same as the direction parallel to the longitudinal direction of the main body portion of the waist support belt.

(Shearing stress of waist support belt)

The maximum shearing stress at the time of bonding the waist support belt main body portion with the hook-and-loop fastener was measured as "shearing stress of waist support belt" in accordance with JIS L 3416 (2010) using Autograph AG-50kNG manufactured by Shimadzu Corporation. Specifically, the waist support belt was bonded in a product state by the female member of the hook-and-loop fastener of the main body portion so as to cover the entire exposed surface of the male member of the waist support belt main body portion, the test piece was attached to the measuring instrument at a distance between chucks of 130 mm, and the test piece was pulled at a pulling speed of 300 mm/min until the bonding surface of the test piece was separated. The same procedure was repeated four times with the same waist support belt, and the maximum stress was measured for each of five times. Here, the "shearing stress of the waist support belt" refers to an average value of five maximum stresses measured by the above method.

(Intrinsic viscosity)

0.8 g of a sample polymer was dissolved in 10 ml of o-chlorophenol (hereinafter, abbreviated as OCP), and the relative viscosity $\eta r$ was determined by the following formula using an Ostwald viscosimeter at a temperature of 25°C to calculate IV.

$$\eta r = \eta/\eta 0 = (t \times d)/(t0 \times d0)$$

$$IV = 0.0242\eta r + 0.2634.$$

In the formula, $\eta$ is the viscosity of the polymer solution, $\eta 0$ is the viscosity of OCP, t is the dropping time of the solution (sec), d is the density of the solution (g/cm$^3$), t0 is the dropping time of OCP (sec), and d0 is the density of OCP (g/cm$^3$).

(Fineness)

The fineness was measured according to JIS L 1013 (2010) 8.3.1 B method. The calculated value of fineness (tex) was multiplied by 10 to obtain fineness (dtex). When the fineness of the fibers contained in the base cloth is measured, the constituent yarns are taken out and measured. Here, when the length of the yarn taken out from the base cloth is less than the predetermined length, the weight (g) per 10,000 m is calculated from the length (m) of the taken out yarn and the weight (g) thereof, and taken as the fineness (dtex).

(Evaluation methods)

(Fastening pressure of waist support belt)

In a test room controlled at a room temperature of 20°C and a relative humidity of 50%, an air-pack pressure measuring system (AMI 3037-5S-II manufactured by AMI) was used to put the waist support belt on a measurement dummy (Dummy Doll KDMD-100 (adult); manufactured by Kato Tech Co., Ltd.), and subjected to measurement. A main body having a length of 100 cm in the longitudinal direction was used for the measurement of the pressure, and the male surface of the hook-and-loop fastener disposed on the first surface and the female surface of the hook-and-loop fastener disposed on the second surface were bonded so that the main body portion overlapped by 16 cm, and then the auxiliary belt was stretched and fastened by 5 cm on each side of left and right. In addition, in the putting on the waist support belt, the back center of the waist support belt was aligned with the back center of the measurement dummy, and the waist support belt was put on so as to be evenly stretched from the back center to the left and right to cover the entire area where the pressure sensor was attached to be described later. In the measurement, the pressure sensors were attached to a total of five positions including two positions on the left and right upper anterior iliac spines on the ventral side of the measurement dummy and three positions on the left and right upper posterior iliac spines on the rear side of the measurement dummy and a midpoint thereof, the garment pressure in the standing posture when the waist support belt was put on the waist of the measurement dummy was measured, and the pressure values were calculated by averaging the pressure values of five positions in total including two positions on the left and right upper anterior iliac spines and three positions on the left and right upper posterior iliac spines on the rear side of the measurement dummy and a midpoint thereof. Here, the "fastening pressure of the waist support belt" refers to an average of the pressure values when the above measurement is repeated three times using the measurement dummy.

(Dimensional change rate and dimensional stability of waist support belt)

Washing was performed 10 times according to JIS L 1096 (2010) F-2 method and 1-2 method in a state where the locking portion of the waist support belt main body portion was locked. A length of the length L1 in the longitudinal direction of the waist support belt main body portion illustrated in Fig. 3 before and after the washing test was performed was measured. A change rate ([(L1 after washing - L1 before washing)/L1 before washing] $\times$ 100 (%)) of the length L1 of the waist support belt main body portion in the longitudinal direction before and after the washing test was performed was obtained, and this was taken as a dimensional change rate (%) of the waist support belt, and the dimensional stability of the waist support belt was evaluated. Here, the "dimensional stability of the waist support belt" was evaluated as "A" when the dimensional change rate of the waist support belt was within $\pm$ 3%, "B" when the dimensional change rate was greater than $\pm$ 3% and within $\pm$ 5%, and "C" when the dimensional change rate was greater than $\pm$ 5%.

(Change in appearance of waist support belt after washing)

As described above, washing was performed 10 times according to JIS L 1096 (2010) F-2 method and 1-2 method in a state where the locking portion of the waist support belt main body portion was locked. Next, the appearance of the waist support belt after washing was checked, whether there was no puckering or ravel was checked, and the change in appearance of the waist support belt after washing was evaluated. Here, the "change in appearance of the waist support belt after washing" was determined by checking the appearance of the waist support belt after washing and integrating the determination results of the grades of puckering and ravel. First, for each of the puckering and ravel, grade determination was performed as "grade 5" when there was no change before and after washing, "grade 4" when slight or partial surface fluffing or puckering was observed after washing, "grade 3" when moderate surface fluffing or puckering was observed after washing, "grade 2" when clear fluffing or puckering was observed after washing, and "grade 1" when dense or excessive fluffing or overall puckering was observed after washing. Here, in a case where the evaluation grade is an intermediate level of each grade, for example, in a case where the evaluation grade is determined to be a level between "grade 4 and grade 5", the evaluation grade is "grade 4-5". The "change in appearance of the waist support belt after washing" was comprehensively evaluated as "A" when the change in appearance was particularly small, "B" when the change in appearance was small, and "C" when the change in appearance was large, from the grade determination results of puckering and ravel.

(Side-by-side composite fiber formed of polytrimethylene terephthalate and polyethylene terephthalate)

Polytrimethylene terephthalate having an intrinsic viscosity of 1.43 and polyethylene terephthalate having an intrinsic viscosity of 0.51 were melted at 285°C and 260°C, respectively, using an extruder, and then subjected to pump weighing so that a composite ratio was polytrimethylene terephthalate: polyethylene terephthalate = 50: 50, and the materials were caused to flow into a spinneret and spun using a composite spinning device to obtain side-by-side type composite fibers having a fineness of 165 dtex and 68 f.

(Examples)

(Example 1)

A warp knitted cloth having a density in the course direction of 42/2.54 cm and a density in the wale direction of 40/2.54 cm was knitted using a 165 dtex polyester-based crimped fiber which is a side-by-side composite fiber formed of polytrimethylene terephthalate and polyethylene terephthalate to obtain a cloth A for a base cloth of 100% by mass polyester.

The crimp appearance of the polyester-based crimped fiber was performed at the stage of finish setting after cloth knitting.

Subsequently, a male member A which was an extrusion type MOLDMAGIC (registered trademark) (manufactured by Kuraray Fastening Co., Ltd.) (made of polyester elastomer, 100% by mass) was obtained as the male member of the hook-and-loop fastener.

Furthermore, a female member A formed of a mesh-like knitted fabric of nylon 100% by mass (nylon 6 fibers) was obtained as a female member of a hook-and-loop fastener. In addition, as an auxiliary material, a flat rubber A, which is a woven fabric including polyester fibers, nylon fibers, and polyurethane elastic fibers, was obtained, and then a waist support belt having an appearance as illustrated in Figs. 1 and 2 was produced using these materials.

Here, the cloth was cut and sewn so that the length direction of the waist support belt and the wale direction of the cloth A were parallel with each other. At this time, a waist support belt having a total length of 100 cm was produced such that, a male member of a hook-and-loop fastener was exposed on the entire surface between a position at 0.10 from a first end portion of a first surface of the main body portion when the length of the waist support belt in the longitudinal direction was set to 1, and the first end portion of the first surface of the main body portion, the female member of the hook-and-loop fastener is exposed on the entire surface between a position at 0.26 from the first end portion of a second surface of the main body portion and the first end portion of the second surface of the main body portion, and the female member of the hook-and-loop fastener was exposed on the entire surface between a position at 0.26 from a second end portion of the second surface of the main body portion and the second end portion of the second surface of the main body portion.

Table 1 shows evaluation results of the first index of the base cloth, the second index of the auxiliary portion, the fastening pressure, and industrial washing durability (dimensional stability, appearance change) for the waist support belt.

(Example 2)

A waist support belt made of the same member as in Example 1 was obtained except that the auxiliary member was a flat rubber B made of nylon fibers and polyurethane elastic fibers. Table 1 shows evaluation results of the first index of the base cloth, the second index of the auxiliary portion, the fastening pressure, and industrial washing durability (dimensional stability, appearance change) for the waist support belt.

(Example 3)

A warp knitted cloth having a density in the course direction of 42/2.54 cm and a density in the wale direction of 30/2.54 cm was knitted using polyurethane elastic fibers of 1240 dtex and nylon fibers of 235 dtex (nylon 6 fibers) to obtain a cloth B for a base cloth of 23% by mass of polyurethane and 77% by mass of nylon. A waist support belt made of the same member as in Example 1 except that the base cloth was the cloth B was obtained. Table 1 shows evaluation results of the first index of the base cloth, the second index of the auxiliary portion, the fastening pressure, and industrial washing durability (dimensional stability, appearance change) for the waist support belt.

(Example 4)

A female member B formed of a plain knitted fabric of nylon 100% by mass (nylon 6 fibers) was obtained as a female member of a hook-and-loop fastener. A waist support belt made of the same member as in Example 1 was obtained except that the female member B was used as the female member of the hook-and-loop fastener. Table 1 shows evaluation results of the first index of the base cloth, the second index of the auxiliary portion, the fastening pressure, and industrial washing durability (dimensional stability, appearance change) for the support belt.

(Example 5)

A warp knitted cloth having a density in the course direction of 42/2.54 cm and a density in the wale direction of 40/2.54 cm was knitted using a 165 dtex polyester-based crimped fiber which is a side-by-side composite fiber formed of polytrimethylene terephthalate and polyethylene terephthalate to obtain a cloth A for a base cloth of 100% by mass polyester.

Subsequently, a male member A which was an extrusion type MOLDMAGIC (registered trademark) (manufactured by Kuraray Fastening Co., Ltd.) (containing 100% by mass of polyester elastomer) was obtained as the male member of the hook-and-loop fastener.

Furthermore, a female member B formed of a plain knitted fabric of nylon 100% by mass (nylon 6 fibers) was obtained as a female member of a hook-and-loop fastener. Further, as an auxiliary member, a flat rubber C which is a knitted fabric of 100% by mass polyester formed of a polyester-based crimped fiber was obtained, and then a waist support belt having an appearance as illustrated in Figs. 1 and 2 was produced using these materials. Here, the cloth was cut and sewn so that the length direction of the waist support belt and the wale direction of the cloth A were parallel with each other. At this time, the configuration of the waist support belt was similar to that in Example 1. Table 1 shows evaluation results of the first index of the base cloth, the second index of the auxiliary portion, the fastening pressure, and industrial washing durability (dimensional stability, appearance change) for the waist support belt.

(Example 6)

As a female member of a hook-and-loop fastener, a female member D made of a woven fabric having a looped surface and containing 100% by mass polyester (polyethylene terephthalate fiber) was obtained. A waist support belt made of the same member as in Example 1 was obtained except that the female member D was used as the female member of the hook-and-loop fastener. Table 2 shows evaluation results of the first index of the base cloth, the second index of the auxiliary portion, the fastening pressure, and industrial washing durability (dimensional stability, appearance change) for the support belt.

(Example 7)

A warp knitted cloth having a density in the course direction of 42/2.54 cm and a density in the wale direction of 40/2.54 cm was knitted using a 165 dtex polyester-based crimped fiber which is a side-by-side composite fiber formed of polytrimethylene terephthalate and polyethylene terephthalate to obtain a cloth A for a base cloth of 100% by mass polyester.

Subsequently, a male member A which was an extrusion type MOLDMAGIC (registered trademark) (manufactured by Kuraray Fastening Co., Ltd.) (100% by mass of polyester elastomer) was obtained as the male member of the hook-and-loop fastener.

Furthermore, as a female member of a hook-and-loop fastener, a female member D made of a woven fabric having a looped surface and containing 100% by mass polyester (polyethylene terephthalate fiber) was obtained. In addition, as an auxiliary material, a flat rubber A, which is a woven fabric including polyester fibers, nylon fibers, and polyurethane elastic fibers, was obtained, and then a waist support belt having an appearance as illustrated in Figs. 1 and 2 was produced using these materials. Here, the cloth was cut and sewn so that the length direction of the waist support belt and the course direction of the cloth A were parallel with each other. At this time, a waist support belt having a total length of 100 cm was produced such that, a male member of a hook-and-loop fastener was exposed on the entire surface between a position at 0.08 from a first end portion of a first surface of the main body portion when the length of the waist support belt in the longitudinal direction was set to 1, and the first end portion of the first surface of the main body portion, the female member of the hook-and-loop fastener is exposed on the entire surface between a position at 0.26 from the first end portion of a second surface of the main body portion and the first end portion of the second surface of the main body portion, and the female member of the hook-and-loop fastener was exposed on the entire surface between a position at 0.26 from a second end portion of the second surface of the main body portion and the second end portion of the second surface of the main body portion. Table 2 shows evaluation results of the first index of the base cloth, the second index of the auxiliary portion, the fastening pressure, and industrial washing durability (dimensional stability, appearance change) for the waist support belt.

(Example 8)

A warp knitted cloth having a density in the course direction of 42/2.54 cm and a density in the wale direction of 40/2.54 cm was knitted using a 330 dtex polyester-based crimped fiber which is a side-by-side composite fiber formed of polytrimethylene terephthalate and polyethylene terephthalate to obtain a cloth E for a base cloth of 100% by mass polyester. A waist support belt was obtained in the same manner as in Example 7 except that the base cloth was the cloth E. Table 2 shows evaluation results of the first index of the base cloth, the second index of the auxiliary portion, the fastening pressure, and industrial washing durability (dimensional stability, appearance change) for the waist support belt.

(Example 9)

A woven cloth having a density in the course direction of 47/2.54 cm and a density in the wale direction of 66/2.54 cm was knitted using an 84 dtex polyester-based crimped fiber which is a side-by-side composite fiber formed of polyethylene terephthalate and polyethylene terephthalate to obtain a cloth F for a base cloth of 100% by mass polyester. A waist support belt was obtained in the same manner as in Example 7 except that the base cloth was the cloth F. Table 2 shows evaluation results of the first index of the base cloth, the second index of the auxiliary portion, the fastening pressure, and industrial washing durability (dimensional stability, appearance change) for the waist support belt.

(Example 10)

A woven cloth having a density in the course direction of 47/2.54 cm and a density in the wale direction of 66/2.54 cm was knitted using an 84 dtex polyester-based crimped fiber which is a side-by-side composite fiber formed of polybutylene terephthalate and polyethylene terephthalate to obtain a cloth G for a base cloth of 100% by mass polyester. A waist support belt was obtained in the same manner as in Example 7 except that the base cloth was the cloth G. Table 2 shows evaluation results of the first index of the base cloth, the second index of the auxiliary portion, the fastening pressure, and industrial washing durability (dimensional stability, appearance change) for the waist support belt.

(Comparative Example 1)

A warp knitted cloth having a density in the course direction of 17/2.54 cm and a density in the wale direction of 20/2.54 cm was knitted using polyester fibers of 700 dtex (polyethylene terephthalate fibers (drawn yarns)) to obtain a cloth C for a base cloth of 100% by mass polyester.
A waist support belt made of the same member as in Example 4 except that the cloth C was used for the base cloth was produced. Table 3 shows evaluation results of the first index of the base cloth, the second index of the auxiliary portion, the fastening pressure, and industrial washing durability (dimensional stability, appearance change) for the waist support belt.

(Comparative Example 2)

A warp knitted cloth having a density in the course direction of 33/2.54 cm and a density in the wale direction of 41/2.54 cm was knitted using nylon fibers of 156 dtex to obtain a cloth D for a base cloth of 100% by mass of nylon.
A waist support belt made of the same member as in Example 4 except that the cloth D was used for the base cloth was produced. Table 3 shows evaluation results of the first index of the base cloth, the second index of the auxiliary portion, the fastening pressure, and industrial washing durability (dimensional stability, appearance change) for the waist support belt.

(Comparative Example 3)

As a female member of a hook-and-loop fastener, a female member C (nylon 71.9% by mass) made of a fabric having a stacking structure of a knitted fabric having a plain surface made of nylon 6 fibers and a polyurethane foam having a thickness of 3 mm was obtained. A waist support belt made of the same member as in Example 1 was produced except that the female member C was used as a female member of a hook-and-loop fastener. Table 3 shows evaluation results of the first index of the base cloth, the second index of the auxiliary portion, the fastening pressure, and industrial washing durability (dimensional stability, appearance change) for the waist support belt.

[Table 1]

| | | | Example 1 | Example 2 | Example 3 | Example 4 | Example 5 |
|---|---|---|---|---|---|---|---|
| Main body portion | | Base cloth | Cloth A | Cloth A | Cloth B | Cloth A | Cloth A |
| | | Male member of hook-and-loop fastener | Male member A | Male member A | Male member A | Male member A | Male member A |
| | | Female member of hook-and-loop fastener | Female member A | Female member A | Female member A | Female member B | Female member B |
| | | Stress at 25% elongation (N/50 mm) of base cloth | 7.8 | 7.8 | 21.0 | 7.8 | 7.8 |
| | | Hysteresis loss rate (%) of base cloth | 24.3 | 24.3 | 23.6 | 24.3 | 24.3 |
| | | Shearing stress (N) of waist support belt | 231.7 | 231.7 | 307.1 | 231.7 | 231.7 |
| | | First index of base cloth | 74.4 | 74.4 | 273.3 | 74.4 | 74.4 |
| Auxiliary portion | | Auxiliary material | Flat rubber A | Flat rubber B | Flat rubber A | Flat rubber A | Flat rubber C |
| | | Stress at 25% elongation (N/50 mm) of the auxiliary portion | 25.2 | 26.4 | 25.2 | 25.2 | 9.2 |
| | | Length of main body portion (cm)/ length of auxiliary portion (cm) | 1.65 | 1.65 | 1.65 | 1.65 | 1.44 |
| | | Hysteresis loss rate (%) of auxiliary portion | 14.1 | 11.9 | 14.1 | 14.1 | 30.5 |
| | | Second index of auxiliary portion | 2.9 | 3.7 | 2.9 | 2.9 | 0.4 |
| Performance | | Fastening pressure of waist support belt (kPa) | 2.6 | 2.4 | 2.4 | 2.7 | 1.7 |
| Industrial washing durability | Dimensional stability of waist support belt after washing | Industrial wash dimensional change rate (%) | -3.1 | -3.1 | -3.6 | -3.5 | -3.5 |
| | | Determination | B | B | B | B | B |
| | Appearance change of waist support belt after industrial washing | Appearance change (ravel) of waist support belt after industrial washing (grade) | 5 | 5 | 4-5 | 5 | 5 |
| | | Appearance change (puckering) of waist support belt after industrial washing (grade) | 4 | 4 | 4 | 4 | 4 |
| | | Comprehensive determination | B | B | B | B | B |

[Table 2]

| | | | Example 6 | Example 7 | Example 8 | Example 9 | Example 10 |
|---|---|---|---|---|---|---|---|
| Main body portion | | Base cloth | Cloth A | Cloth A | Cloth E | Cloth F | Cloth G |
| | | Male member of hook-and-loop fastener | Male member A | Male member A | Male member A | Male member A | Male member A |
| | | Female member of hook-and-loop fastener | female member D | female member D | female member D | female member D | female member D |
| | | Stress at 25% elongation (N/50 mm) of base cloth | 7.8 | 15.2 | 29.7 | 9.2 | 15.6 |
| | | Hysteresis loss rate (%) of base cloth | 24.3 | 41.4 | 29.5 | 38.5 | 34.4 |
| | | Shearing stress (N) of waist support belt | 349.5 | 391.3 | 313.7 | 276.6 | 386.9 |
| | | First index of base cloth | 112.2 | 143.7 | 225.0 | 65.8 | 208.2 |
| Auxiliary portion | | Auxiliary material | Flat rubber A | Flat rubber A | Flat rubber A | Flat rubber A | Flat rubber A |
| | | Stress at 25% elongation (N/50 mm) of the auxiliary portion | 25.2 | 25.2 | 25.2 | 25.2 | 25.2 |
| | | Length of main body portion (cm)/ length of auxiliary portion (cm) | 1.65 | 1.65 | 1.65 | 1.65 | 1.65 |
| | | Hysteresis loss rate (%) of auxiliary portion | 14.1 | 14.1 | 14.1 | 14.1 | 14.1 |
| | | Second index of auxiliary portion | 2.9 | 2.9 | 2.9 | 2.9 | 2.9 |
| Performance | | Fastening pressure of waist support belt (kPa) | 2.8 | 2.8 | 2.8 | 2.6 | 2.7 |
| Industrial washing durability | Dimensional stability of waist support belt after washing | Industrial wash dimensional change rate (%) | -2.9 | -2.6 | -2.7 | -3.5 | -3 |
| | | Determination | A | A | A | B | B |
| | Appearance change of waist support belt after industrial washing | Appearance change (ravel) of waist support belt after industrial washing (grade) | 5 | 5 | 5 | 5 | 5 |
| | | Appearance change (puckering) of waist support belt after industrial washing (grade) | 4-5 | 4-5 | 4-5 | 4 | 4-5 |
| | | Comprehensive determination | A | A | A | B | A |

[Table 3]

| | | | Comparative Example 1 | Comparative Example 2 | Comparative Example 3 |
|---|---|---|---|---|---|
| Main body portion | | Base cloth | Cloth C | Cloth D | Cloth A |
| | | Male member of hook-and-loop fastener | Male member A | Male member A | Male member A |
| | | Female member of hook-and-loop fastener | Female member B | Female member B | Female member C |
| | | Stress at 25% elongation (N/50 mm) of base cloth | 9.2 | 49.5 | 7.8 |
| | | Hysteresis loss rate (%) of base cloth | 30.5 | 38.4 | 24.3 |
| | | Shearing stress (N) of waist support belt | 187.2 | 325.7 | 200.6 |
| | | First index of base cloth | 56.5 | 420 | 64.4 |
| Auxiliary portion | | Auxiliary material | Flat rubber A | Flat rubber A | Flat rubber A |
| | | Stress at 25% elongation (N/50 mm) of the auxiliary portion | 25.2 | 25.2 | 25.2 |
| | | Length of main body portion (cm)/ length of auxiliary portion (cm) | 1.65 | 1.65 | 1.65 |
| | | Hysteresis loss rate (%) of auxiliary portion | 14.1 | 14.1 | 14.1 |
| | | Second index of auxiliary portion | 2.9 | 2.9 | 2.9 |
| Performance | | Fastening pressure of waist support belt (kPa) | 1.3 | 2.6 | 2.6 |
| Industrial washing durability | Dimensional stability of waist support belt after washing | Industrial wash dimensional change rate (%) | -4.9 | -6.6 | -6.4 |
| | | Determination | B | C | C |
| | Appearance change of waist support belt after industrial washing | Appearance change (ravel) of waist support belt after industrial washing (grade) | 5 | 4 | 5 |
| | | Appearance change (puckering) of waist support belt after industrial washing (grade) | 4 | 2-3 | 2 |
| | | Comprehensive determination | B | C | c |

DESCRIPTION OF REFERENCE SIGNS

1:    Main body portion

2:    Auxiliary portion

3:    Male member of hook-and-loop fastener

4:    Female member of hook-and-loop fastener

5:    Base cloth

6:    Tape

7:    Bone

8:    Piping tape

9:    Perforation

**Claims**

1.  A waist support belt comprising

a main body portion having a band shape,
wherein the main body portion includes a base cloth, a male member of a hook-and-loop fastener, and a female member of a hook-and-loop fastener,
the male member of the hook-and-loop fastener is sewn to the base cloth at a first end portion of a first surface of the main body portion,
the female member of the hook-and-loop fastener is sewn to the base cloth at both end portions of a second surface of the main body portion,
the male member of the hook-and-loop fastener contains a polyester elastomer resin in an amount of 95% by mass or more with respect to a mass of the male member of the hook-and-loop fastener,
the female member of the hook-and-loop fastener contains one or more resins selected from the group consisting of a polyester resin and a polyamide resin in an amount of 95% by mass or more with respect to a mass of the female member of the hook-and-loop fastener,
the base cloth is formed of one or more fibers selected from the group consisting of a polyester crimped fiber and a polyurethane elastic fiber, and
a first index of the base cloth [stress at 25% elongation (N/50 mm) of base cloth/hysteresis loss rate (%)] $\times$ [shearing stress (N) of waist support belt] is 60 to 300.

2.  The waist support belt according to claim 1, wherein

the male member of the hook-and-loop fastener is exposed on an entire surface between a position at 0.05 to 0.2 from the first end portion of the first surface of the main body portion when a length, the first surface of the main body portion, in a longitudinal direction of the main body portion is set to 1 and the first end portion of the first surface of the main body portion,
the female member of the hook-and-loop fastener is exposed on an entire surface between a position at 0.2 to 0.3 from a first end portion of the second surface of the main body portion when the length, the second surface of the main body portion, in the longitudinal direction of the main body portion is set to 1 and the first end portion of the second surface of the main body portion, and
the female member of the hook-and-loop fastener is exposed on the entire surface between a position of 0.2 to 0.3 from a second end portion of the second surface of the main body portion and the second end portion of the second surface of the main body portion when the length, the second surface of the main body portion, in the longitudinal direction of the main body portion is set to 1, and the second end portion of the second surface of the main body portion.

3.  The waist support belt according to claim 1 or 2, further comprising

an auxiliary portion having a band shape,
wherein the male member of the hook-and-loop fastener is located at both end portions of one surface of the auxiliary portion,
a central portion of the auxiliary portion in a longitudinal direction of the auxiliary portion is sewn to the central portion of the main body portion in the longitudinal direction of the main body portion, and
a second index of the auxiliary portion [stress at 25% elongation (N/50 mm) of the auxiliary portion $\times$ length (cm) of the main body]/[length (cm) of the auxiliary portion $\times$ hysteresis loss rate (%)] is 2.5 to 4.0.

4.  The waist support belt according to any one of claims 1 to 3, wherein the base cloth is a knitted fabric.

**5.** The waist support belt according to any one of claim 1 to 4, wherein the base cloth is formed of a polyester crimped fiber.

**6.** The waist support belt according to any one of claims 1 to 5, wherein the polyester crimped fiber is a bimetal composite fiber in which two types of polyester polymers having a difference in intrinsic viscosity of 0.30 or more are bonded to each other in a side-by-side type along a fiber length direction.

**7.** The waist support belt according to claim 6, wherein the two types of polyester polymers are two types of polyester polymers selected from polyethylene terephthalate, polytrimethylene terephthalate, and polybutylene terephthalate.

**8.** The waist support belt according to any one of claims 1 to 7, wherein the polyester crimped fiber is a side-by-side type composite fiber formed of polytrimethylene terephthalate and polyethylene terephthalate.

**9.** The waist support belt according to any one of claims 1 to 8, wherein the polyester crimped fiber has a fineness of 100 to 400 dtex.

Fig. 1

Fig. 2

Fig. 3

Fig. 4

## INTERNATIONAL SEARCH REPORT

| | International application No. |
|---|---|
| | PCT/JP2021/004572 |

A. CLASSIFICATION OF SUBJECT MATTER
A44B 18/00(2006.01)i; A41C 1/00(2006.01)i; A41C 1/02(2006.01)i; A41D 13/05(2006.01)i
FI: A41D13/05 125; A41C1/02 A; A41C1/00 A; A44B18/00
According to International Patent Classification (IPC) or to both national classification and IPC

B. FIELDS SEARCHED

Minimum documentation searched (classification system followed by classification symbols)
A44B18/00; A41C1/00; A41C1/02; A41D13/05

Documentation searched other than minimum documentation to the extent that such documents are included in the fields searched
Published examined utility model applications of Japan   1922-1996
Published unexamined utility model applications of Japan   1971-2021
Registered utility model specifications of Japan   1996-2021
Published registered utility model applications of Japan   1994-2021

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2014-46138 A (GUNZE LIMITED) 17 March 2014 (2014-03-17) entire text, all drawings | 1-8 |
| A | JP 2018-76628 A (MIZUNO CORPORATION) 17 May 2018 (2018-05-17) entire text, all drawings | 1-8 |
| A | JP 2003-245108 A (KURARAY CO., LTD.) 02 September 2003 (2003-09-02) entire text, all drawings | 1-8 |
| A | JP 2016-521598 A (GOTTLIEB BINDER GMBH & CO. KG) 25 July 2016 (2016-07-25) entire text, all drawings | 1-8 |
| A | JP 2018-197041 A (TEIJIN LTD.) 13 December 2018 (2018-12-13) entire text, all drawings | 1-8 |

☒ Further documents are listed in the continuation of Box C.    ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 19 March 2021 (19.03.2021) | 06 April 2021 (06.04.2021) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2021/004572

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2003-325208 A (ASAHI KASEI CORPORATION) 18 November 2003 (2003-11-18) entire text, all drawings | 1-8 |

Form PCT/ISA/210 (continuation of second sheet) (January 2015)

**INTERNATIONAL SEARCH REPORT**

Information on patent family members

| International application No. |
| --- |
| PCT/JP2021/004572 |

| Patent Documents referred in the Report | Publication Date | Patent Family | Publication Date |
| --- | --- | --- | --- |
| JP 2014-46138 A | 17 Mar. 2014 | (Family: none) | |
| JP 2018-76628 A | 17 May 2018 | (Family: none) | |
| JP 2003-245108 A | 02 Sep. 2003 | US 2003/0186021 A1 entire text, all drawings EP 1338212 A2 CN 1440702 A KR 10-0484575 B1 | |
| JP 2016-521598 A | 25 Jul. 2016 | US 2016/0106185 A1 entire text, all drawings WO 2014/198381 A1 CN 105283092 A KR 10-2016-0019061 A | |
| JP 2018-197041 A | 13 Dec. 2018 | (Family: none) | |
| JP 2003-325208 A | 18 Nov. 2003 | (Family: none) | |

Form PCT/ISA/210 (patent family annex) (January 2015)

**EP 4 104 703 A1**

## REFERENCES CITED IN THE DESCRIPTION

**Patent documents cited in the description**

- JP 2017179663 A

- JP H8182794 A